Europäisches Patentamt

European Patent Office

Office européen des brevets

(19)

(11) Publication number: **0 026 041**
**B1**

(12) **EUROPEAN PATENT SPECIFICATION**

(45) Date of publication of patent specification: **26.01.83**

(21) Application number: **80302762.2**

(22) Date of filing: **12.08.80**

(51) Int. Cl.³: **C 07 C 41/06,**
C 07 C 43/04,
C 07 C 11/02, C 07 C 6/00,
C 10 G 35/095,
B 01 J 29/28

(54) **A method for producing olefins and/or ethers of high octane number.**

(30) Priority: **30.08.79 US 71426**

(43) Date of publication of application:
**01.04.81 Bulletin 81/13**

(45) Publication of the grant of the patent:
**26.01.83 Bulletin 83/4**

(84) Designated Contracting States:
**BE DE FR GB IT NL**

(56) References cited:
**DD - A - 133 661**
**US - A - 3 832 449**
**US - A - 4 071 573**

(73) Proprietor: **MOBIL OIL CORPORATION**
**150 East 42nd Street**
**New York New York 10017 (US)**

(72) Inventor: **Garwood, William Everett**
**125 Warwick Road**
**Haddonfield New Jersey (US)**
Inventor: **Schoennagel, Hans-Juergen**
**42 Pine Knoll Drive**
**Lawrenceville New Jersey (US)**

(74) Representative: **Cooper, John Anthony**
**Mobil Court 3 Clements Inn**
**London WC2A 2EB (GB)**

Courier Press, Leamington Spa, England.

# 0 026 041

## A method for producing olefins and/or ethers of high octane number

There are several unit processes in a petroleum refinery which produce, by design or inherently a mixture of gaseous and liquid olefins, for example $C_2$ plus olefins and particularly a mixture of $C_2$ to $C_{10}$ olefins. These olefinic products may comprise some paraffinic components mixed therewith. The prior art is replete with processes for converting such olefinic products before and after separation of paraffinic components from olefinic components. For example, components of these olefinic materials may be subjected to alkylation, polymerization, oligomerization, dimerization and aromatization. U.S. Patent 3,760,002 discloses the aromatization of feedstocks comprising $C_2$ to $C_4$ paraffins and olefins with a crystalline zeolite having the characteristics of ZSM-5 crystalline zeolite. U.S. Patent 3,756,942 discloses the conversion of feedstock boiling in the range of $C_5$ hydrocarbons up to about 250°F (121°C) over a ZSM-5 crystalline zeolite to form aromatics. U.S. Patent 3,960,978 discloses the conversion of $C_2$ to $C_5$ olefins, alone or in admixture with paraffins, into an olefinic gasoline by passage over a ZSM-5 crystalline zeolite of controlled acidity. In this patented process, the crystalline zeolite alpha ($\alpha$) value is maintained within the range of 0.1 to about 120, the hydrocarbon partial pressure is within the range of $5.1 \times 10^4$ Pa to $4.05 \times 10^6$ Pa (0.5 to 40 atmospheres) using a temperature within the range of 260°C (500°F) to 482°C (900°F) to produce oligomers of the olefin feed and comprising $C_9$ product material.

It is known that isobutylene may be reacted with methanol over an acidic catalyst to produce methyl tertiary butyl ether (MTBE) and isoamylenes may be reacted with methanol over an acidic catalyst to produce tertiary amyl methyl ether (TAME). The catalyst employed is preferably an ion exchange resin in the hydrogen form. Substantially any acidic catalyst may be employed with varying degrees of success.

That is, acidic solid catalyst may be used such as sulfonic resins, phosphoric acid modified kieselguhr, silica alumina and acid zeolites.

The present invention is concerned with producing high octane ethers TAME and MTBE for admixture with gasoline from a mixture of olefins and preferably a mixture of $C_2$ to $C_{10}$ olefins under particularly selected conditions in the presence of catalyst suitable for the purpose. The novel processing combination of the present invention departs significantly from the prior art and initially involves a particular restructuring or rearrangement of the wide olefin composition stream available as charge to provide high yields of tertiary $C_4$ and $C_5$ olefin components.

Accordingly, the invention resides in one aspect in a method for producing gasoline boiling range high octane products from a mixture of olefins comprising up to $C_{10}$ olefins which comprises, contacting a mixed olefin feed with an acid crystalline zeolite catalyst characterized by a pore opening of at least $5 \times 10^{-10}$ m, a silica to alumina ratio of at least 12 and a constraint index within the range of 1 to 12 at an olefin pressure below $5.1 \times 10^4$ Pa (0.5 atmosphere) and a temperature selected from within the range of 204°C (400°F) to 316°C (600°F), and recovering an olefinic product enriched with $C_4$ and $C_5$ olefins comprising olefin isomers.

The conversion of the olefin feed into the product enriched with $C_4$ and $C_5$ olefins comprising olefin isomers involves a combination of operations including cracking, polymerization or dimerization and modification of the olefin feed chain length to provide a product composition other than multiples of carbon atom chain growth of the individual olefins in the feed such as is normally obtained by polymerization and/or dimerization. For lack of better identification, applicants refer to the reactions encountered in the initial olefin restructing operation as one of olefination. The restructuring of the broad carbon chain olefin charge stream according to this invention produces a mixture of $C_4$ to $C_7$ olefins, preferentially with the major portion being $C_4$ and $C_5$ olefins comprising a high percentage of tertiary olefins such as isobutylene and isoamylenes and less than 15% by weight of $C_9$ and higher boiling olefins. The initial olefin conversion or olefination step is therefore carried out under conditions to particularly produce high yields of isobutylene and tertiary isopentenes, which material can thereafter be converted to desired $C_4$ and $C_5$ high octane methyl ethers.

Preferably, the olefin feed is contacted with said crystalline zeolite at a temperature within the range of 260°C (500°F) to 288°C (550°F) at an olefin pressure not above $3 \times 10^4$ Pa (0.3 atmosphere) and sufficient to obtain 95% conversion to a higher octane product comprising less than 2% aromatics. Conveniently the olefin pressure is as low as about $7 \times 10^3$ Pa (1 psia).

Preferably, a diluent material substantially inert to the restructuring of the olefins charged is employed with said olefin feed to maintain the partial pressure of the olefin feed below $5.1 \times 10^4$ Pa (0.5 atmosphere).

Conveniently, the diluent material comprises one or more of hydrogen, propane and nitrogen.

Conveniently, the olefin restructuring operation of the present invention is performed with WHSV (weight hourly space velocity) of the olefin feed being maintained within the range of 0.2 to 5. Moreover, the acid crystalline zeolite restructuring catalyst may be combined with a binder, for example, to form an extrudate.

Preferably, the olefinic product of the restructuring operation comprises isobutylene and isopentenes and these material are reacted with an alcohol by contact with an acidic catalyst to form high octane ethers.

In a further aspect, the invention resides in a method for producing high octane ether products comprising tertiary amyl methyl ether and methyl tertiary butyl ether from a $C_2$ to $C_{10}$ olefin feed mixture which comprises, reacting the $C_2$ to $C_{10}$ olefin feed under temperature and pressure conditions in the presence of an acid zeolite catalyst providing a pore opening of at least $5 \times 10^{-10}$m, a silica-alumina ratio of at least 12 and a constraint index within the range of 1 to 12 to form an olefin product richer in $C_4$ and $C_5$ olefins and comprising olefin isomers of isobutylene and isopentenes, reacting at least the olefin isomers with a lower alcohol in the presence of an acid catalyst under temperature and pressure conditions promoting the formation of the high octane ethers above identified, separating the formed ethers from unreacted $C_4$ minus olefins and alcohol and recovering the separated ethers, and recycling the separated $C_4$ minus olefins and alcohol for contact with the zeolite catalyst above identified.

Preferably the formation of ethers from $C_4$ and $C_5$ olefin isomers is accomplished with methanol and an acidic resin catalyst.

Preferably, $C_5$ plus olefins are recovered with the ether product. Olefins boiling below $C_6$ olefins are preferably separated from the ether product and recycled to the zeolite catalyst conversion step.

Conveniently, the olefin product of the zeolite catalyst conversion operation is separated to recover particularly $C_4$ to $C_7$ olefins which are thereafter reacted with methanol in the presence of a solid acidic catalyst to form the ether products desired and unreacted olefins are separated from the ether product to provide separate streams thereof for use in blending to form desired gasoline product.

In yet a further aspect, the invention resides in a method for producing high octane ethers and olefins from a mixture of $C_2$ to $C_{10}$ olefins which comprises, converting the $C_2$ to $C_{10}$ olefins in the presence of a special zeolite catalyst by olefination thereof to a product enriched in $C_4$ to $C_7$ olefins, reacting the $C_4$ to $C_7$ olefins thus produced with methanol and in the presence of an acid catalyst to form ethers of isobutylene and isopentenes, separating the ether product and $C_6$ plus olefins from unreacted $C_5$ minus olefins and methanol, and recycling the $C_5$ minus olefins with methanol to the special zeolite catalyst olefination operation, said special zeolite catalyst comprising a crystalline zeolite characterized by a pore opening of at least $5 \times 10^{-10}$ m, a silica to alumina ratio of at least 12 and a constraint index within the range of 1 to 12. The crystalline aluminosilicates herein referred to, also known as zeolites, constitute an unusual class of natural and synthetic minerals. They are characterized by having a rigid crystalline framework structure composed of an assembly of silicon and aluminum atoms, each surrounded by a tetrahedron of shared oxygen atoms, and a precisely defined pore structure. Exchangeable cations are present in the pores.

The catalysts referred to herein utilize members of a special class of zeolites exhibiting some unusual properties. These zeolites can induce profound transformations of aliphatic hydrocarbons to aromatic hydrocarbons in commercially desirable yields and are generally highly effective in alkylation, isomerization, disproportionation and other reactions involving aromatic hydrocarbons. Although they may have unusually low alumina contents, i.e. high silica to alumina ratios, they are very active even with silica to alumina ratios exceeding 30. This activity is surprising, since catalytic activity of zeolites is generally attributed to framework aluminum atoms and cations associated with these aluminum atoms. These zeolites retain their crystallinity for long periods in spite of the presence of steam even at high temperatures which induce irreversible collapse of the crystal framwork of other zeolites, e.g. of the X and A type. Furthermore, carbonaceous deposits, when formed, may be removed by burning at higher than usual temperatures to restore activity. In many environments the zeolites of this class exhibit very low coke forming capability, conducive to very long times on stream between burning regenerations.

An important characteristic of the crystal structure of this class of zeolites is that it provides constrained access to, and egress from, the intracrystalline free space by virtue of having a pore dimension greater than about $5 \times 10^{-10}$ m and pore windows of about a size such as would be provided by 10-membered rings of oxygen atoms. It is to be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. Briefly, the preferred zeolites useful in this invention possess, in combination: a silica to alumina ratio of at least about 12; and a structure providing constrained access to the crystalline free space.

The silica to alumina ratio referred to may be determined by conventional analysis. This ratio is meant to represent, as closely as possible, the ratio in the rigid anionic framework of the zeolite crystal and to exclude aluminum in the binder or in cationic or other form within the channels. Although zeolites with a silica to alumina ratio of at least 12 are useful, it is preferred to use zeolites having higher ratios of at least about 30. Such zeolites, after activation, acquire an intracrystalline sorption capacity for normal hexane which is greater than that for water, i.e., they exhibit "hydrophobic" properties. It is believed that this hydrophobic character is advantageous in the present invention.

The zeolites useful as catalysts in this invention freely sorb normal hexane and have a pore dimension greater than about $5 \times 10$ m (5 Angstroms). In addition, their structure must provide constrained access to some larger molecules. It is sometimes possible to judge from a known crystal

3

0 026 041

structure whether such constrained access exists. For example, if the only pore windows in a crystal are formed by 8-membered rings, then access by molecules of larger cross-section than normal hexane is substantially excluded and the zeolite is not of the desired type. Zeolites with windows of 10-membered rings are preferred, although excessive puckering or pore blockage may render these zeolites substantially ineffective. Zeolites with windows of 12-membered rings do not generally appear to offer sufficient constraint to produce the advantageous conversions desired in the instant invention, although structures can be conceived, due to pore blockage or other cause, that may be operative.

Rather than attempt to judge from crystal structure whether or not a zeolite possesses the necessary constrained access, a simple determination of the "constraint index" may be made by continuously passing a mixture of equal weight of normal hexane and 3-methylpentane over a small sample, approximately 1 gram or less, of zeolite at atmospheric pressure according to the following procedure. A sample of the zeolites, in the form of pellets or extrudate, is crushed to a particle size about that of coarse sand and mounted in a glass tube. Prior to testing, the zeolite is treated with a stream of air at 1000°F (538°C) for at least 15 minutes. The zeolite is then flushed with helium and the temperature adjusted between 550°F (288°C) and 950°F (510°C) to give an overall conversion between 10% and 60%. The mixture of hydrocarbons is passed at 1 liquid hourly space velocity (i.e., 1 volume of liquid hydrocarbon per volume of catalyst per hour) over the zeolite with a helium dilution to give a helium to total hydrocarbon mole ratio of 4:1. After 20 minutes on stream, a sample of the effluent is taken and analyzed, most conveniently by gas chromatography, to determine the fraction remaining unchanged for each of the two hydrocarbons.

The "constraint index" is calculated as follows:

$$\text{Constraint index} = \frac{\log 10 \text{ (fraction of n-hexane remaining)}}{\log 10 \text{ (fraction of 3-methylpentane remaining)}}$$

The constraint index approximates the ratio of the cracking rate constants for the two hydrocarbons. Catalysts suitable for the present invention are those which employ a zeolite having a constraint index from 1.0 to 12.0. Constraint idex (C.I.) values for some typical zeolites, including some not within the scope of this invention, are:

| CAS | C.I. |
| --- | --- |
| ZSM-5 | 8.3 |
| Erionite | 38 |
| ZSM-11 | 8.7 |
| ZSM-35 | 6.0 |
| TMA Offretite | 3.7 |
| ZSM-38 | 2.0 |
| ZSM-12 | 2. |
| Beta | 0.6 |
| ZSM-4 | 0.5 |
| Acid mordenite | 0.5 |
| REY | 0.4 |
| Amorphous silica-alumina | 0.6 |

The above-described constraint index is an important, and even critical, definition of those zeolites which are useful to catalyze the instant process. The very nature of this parameter and the recited technique by which it is determined, however, admit of the possibility that a given zeolite can be tested under somewhat different conditions and thereby have different constraint indexes. Constraint index seems to vary somewhat with severity of operation (conversion). Therefore, it will be appreciated that it may be possible to so select test conditions to establish multiple constraint indexes for a particular given zeolite which may be both inside and outside the above defined range of 1 to 12.

Thus, it should be understood that the parameter and property "Constraint Index" as such value is used herein is an inclusive rather than an exclusive value. That is, a zeolite when tested by any combination of conditions within the testing definition set forth hereinabove and to have a constraint index of 1 to 12 is intended to be included in the instant catalyst definition regardless that the same identical zeolite tested under other defined conditions may give a constraint index value outside of 1 to 12.

The class of zeolites defined herein is exemplified by ZSM-5, ZSM-11, ZSM-12, ZSM-35 and ZSM-38, and other similar materials. Recently issued U.S. Patent 3,702,886, describing and claiming ZSM-5 is incorporated herein by reference.

ZSM-11 is more particularly described in U.S. Patent 3,709,979, the entire contents of which are incorporated herein by reference.

ZSM-12 is more particularly described in U.S. Patent 3,832,449, the entire contents of which are incorporated herein by reference.

4

The subject of ZSM-35 is described in U.S. Application Serial No. 528,061 filed November 29, 1974. The subject of ZSM-38 is described in U.S. Application Serial No. 528,060 filed November 29, 1974.

The specific zeolites described, when prepared in the presence of organic cations, are substantially catalytically inactive, possibly because the intracrystalline free space is occupied by organic cations from the forming solution. They may be activated by heating in an inert atmosphere at 1000°F (538°C) for 1 hour, for example, followed by base exchange with ammonium salts followed by calcination at 1000°F (538°C) in air. The presence of organic cations in the forming solution may not be absolutely essential to the formation of this special type zeolite; however, the presence of these cations does not appear to favor the formation of this special type of zeolites. More generally, it is desirable to activate this type zeolite by base exchange with ammonium salts followed by calcination in air at about 1000°F (538°C) for from about 15 minutes to about 24 hours.

Natural zeolites may sometimes be converted to this type zeolite by various activation procedures and other treatments such as base exchange, steaming, alumina extraction and calcination, alone or in combinations. Natural minerals which may be so treated include ferrierite, brewsterite, stilbite, dachiardite, epistilbite, heulandite and clinoptilolite. The preferred crystalline aluminosilicates are ZSM-5, ZSM-11, ZSM-12, ZSM-35 and ZSM-38, with ZSM-5 particularly preferred.

The zeolites used as catalysts in this invention may be in the hydrogen form or they may be base exchanged or impregnated to contain ammonium or a metal cation complement. It is desirable to calcine the zeolite after base exchange. The metal cations that may be present include any of the cations of the metals of Groups I through VIII of the Periodic Table. However, in the case of Group IA metals, the cation content should in no case be so large as to substantially eliminate the activity of the zeolite for the catalysis being employed in the instant invention. For example, a completely sodium exchanged H-ZSM-5 appears to be largely inactive for shape selective conversions required in the present invention.

In a preferred aspect of this invention, the zeolites useful as catalysts herein are selected as those having a crystal framework density, in the dry hydrogen form, of not substantially below about 1.6 grams per cubic centimeter. It has been found that zeolites which satisfy all three of these criteria are most desired. Therefore, the preferred catalysts of this invention are those comprising zeolite having a constraint index as defined above of about 1 to 12, a silica to alumina ratio of at least about 12 and a dried crystal density of not substantially less than about 1.6 grams per cubic centimeter. The dry density for known structures may be calculated from the number of silicon plus aluminum atoms per $10^{-27}$ m$^3$ as given, e.g., on page 19 of the article on Zeolite Structure by W. M. Meier. This paper, the entire contents of which are incorporated herein by reference, is included in "Proceedings of the Conference on Molecular Sieves, London, April 1967", published by the Society of Chemical Industry, London, 1968. When the crystal structure is unknown, the crystal framework density may be determined by classical pycnometer techniques. For example, it may be determined by immersing the dry halogen form of the zeolite in an organic solvent which is not sorbed by the crystal. It is possible that the unusual sustained activity and stability of this class of zeolites are associated with its high crystal anionic framework density of not less than about 1.6 grams per cubic centimeter. This high density of course must be associated with a relatively small amount of free space within the crystal, which might be expected to result in more stable structures. This free space, however, seems to be important as the locus of catalytic activity.

Crystal framework densities of some typical zeolites, including some which are not within the purview of this invention, are:

| Zeolite | Void volume | Framework density |
|---|---|---|
| Ferrierite | 0.28 cc/cc | 1.76 g/cc |
| Mordenite | .28 | 1.7 |
| ZSM-5, -11 | .29 | 1.79 |
| Dachiardite | .32 | 1.72 |
| L | .32 | 1.61 |
| Clinoptilolite | .34 | 1.71 |
| Laumontite | .34 | 1.77 |
| ZSM-4 (Omega) | .38 | 1.65 |
| Heulandite | .39 | 1.69 |
| P | .41 | 1.57 |
| Offretite | .40 | 1.55 |
| Levynite | .40 | 1.54 |
| Erionite | .35 | 1.51 |
| Gmelinite | .44 | 1.46 |
| Chabazite | .47 | 1.45 |
| A | .5 | 1.3 |
| Y | .48 | 1.27 |

An intimate mixture of heterogeneous catalysts may be prepared in various ways. The two components may be separately prepared in the form of catalyst particles such as pellets or extrudates, for example, and simply mixed in the required proportions. The particle size of the individual component particles may be quite small, for example from about 20 to 150 microns, when intended for use in fluid bed operation; or they may be as large as up to about 1/2 inch (1.25 cm) for fixed bed operation. Or, the two components may be mixed as powders and formed into pellets or extrudate, each pellet containing both components in substantially the required proportions. Binders such as alumina, zirconia, silica, titania, magnesia, etc., may be present. Base exchange of the crystalline aluminosilicate component may be used in some selected cases to effect the introduction of part or all of a metal component. Other means for forming the intimate mixture may be used, such as: precipitation of the metal component in the presence of the acidic crystalline aluminosilicate; or electroless deposition of metal on the zeolite; or deposition of metal from the vapor phase. Various combinations of the above preparative methods will be obvious to those skilled in the art of catalyst preparation. It should be cautioned, however, to avoid techniques likely to reduce the crystallinity of the acidic crystalline aluminosilicate.

Figure 1 is a graph showing the effect of olefin feed partial pressure on product composition.

Figure 2 is a graph showing the liquid product boiling point and the $C_5$ plus octane rating obtained when converting propylene.

Figure 3 is a graph showing a comparison between observed and calculated octane of olefination product+a light reformate.

Figure 4 is a diagrammatic sketch of the processing arrangement of the present invention.

A distillation of the liquid products obtained from propylene olefination alone at 96.5 kPa (14 psia) and propylene diluted with nitrogen ratio (1.10) to reduce the propylene to 8.96 kPa (1.3 psia) shows in Table 1 below a substantial shift in composition and thus boiling points. The same components are present, but shifted substantially in concentration to a lower carbon number product, as shown in the table below:

TABLE 1

Component breakdown of $C_5+$

|  | $C_3=$Alone | 10/1 $N_2C_3=$ |
|---|---|---|
| n-$C_5$ | 0.3 | 0.2 |
| i-$C_5$ | 1.3 | 0.6 |
| $C_5=$ | 16.9 | 36.8 |
| Cyclo $C_5$ | 0.1 | — |
| n-$C_6$ | 0.4 | 0.2 |
| i-$C_6$ | 1.5 | 0.7 |
| $C_6=$ | 15.2 | 22.9 |
| Cyclo $C_6$ | .1 | — |
| n-$C_7$ | 0.3 | 0.1 |
| i-$C_7$ | 1.5 | 0.6 |
| $C_7=$ | 17.2 | 20.3 |
| DiMe-$N_5$ | — | 0.1 |
| n-$C_8$ | 0.2 | 0.1 |
| Iso-$C_8$—P+O+$N_5$+$N_6$ | 14.9 | 10.4 |
| n-$C_9$ | 0.7 | 0.1 |
| Iso-$C_9$—P+O+$N_5$+$N_6$ | 12.9 | 5.1 |
| n-$C_{10}$ | 0.3 | — |
| Iso-$C_{10}$—P+O+$N_5$+$N_6$ | 7.8 | 1.5 |
| Unknown | 8.4 | 0.3 |
|  | 100.01 | 100.0 |

The low pressure olefination operation of this invention provides a larger proportion of shorter chain olefin components of improved quality as represented by octane number and lower total product end point than obtained when processing the olefin feed under the higher pressure condition. In order to obtain a better understanding of the low pressure operation of this invention, three different olefin charge materials were contacted under particularly selected operating conditions. That is, to better understand the conversion potential of components of a broad olefin charge, propylene, a pentene mixture and 1-hexene were separately converted as herein defined. In the following examples, the three separate feeds were admixed with hydrogen in a 10/1 $H_2$/HC ratio for contact at atmospheric pressure and a temperature of about 530°F (277°C) with an acidic ZSM-5 crystalline zeolite catalyst. A weight hourly space velocity (WHSV) in the range of 0.5—0.9 was employed. The results obtained are provided in Table 2 below.

6

## TABLE 2

### Carbon number distribution

Atmospheric pressure, 10/1 $H_2$/HC 530°F (277°C), 0.5—0.9 WHSV

| Example | 1 | 2 | 3 |
|---|---|---|---|
| Charge | Propylene | Pentene mixture | 1-Hexane |
| Product | | | |
| $C_1+C_2$, Wt.% | <0.1 | <0.1 | 0.1 |
| $C_3$ | 9.5 | 10.1 | 8.9 |
| $C_4$ | 26.7 | 20.0 | 20.0 |
| $C_5$ | 29.3 | 26.4 | 22.4 |
| $C_6$ | 13.3 | 15.1 | 16.0 |
| $C_7$ | 11.1 | 11.2 | 10.3 |
| $C_8$ | 5.7 | 7.0 | 8.0 |
| $C_9$ | 2.9 | 5.3 | 6.4 |
| $C_{10}+$ | 1.5 | 4.9 | 7.5 |

It will be observed upon examination of the data of Table 2 that the three separate feeds vary considerably in carbon number but each produces close to equilibrium conversion mixtures rich in $C_4$ and $C_5$ olefins. Some $C_6$ and $C_7$ olefins are also produced, with $C_5$ carbon compounds predominating. The $C_5$ product mixture from each feed was separately examined. It was found that about 80% of the $C_5$ olefins are tertiary olefins as summarized below in Table 3.

## TABLE 3

### $C_5$ Olefin distribution

| | 1 | 2 | | 3 | |
|---|---|---|---|---|---|
| | Propylene | Pentenes | | 1-Hexene | |
| Example charge | Product $C_5$ analysis | Charge (a) | Product $C_5$ analysis | Product $C_5$ analysis | Equilibrium values |
| $C_5=1$ | 2 | <1.0% | 2 | 2 | 2 |
| $2M1C_4=$ | 16 | 8.0% | 18 | 18 | 20 |
| $3M1C_4=$ | 2 | <1.0% | 2 | 2 | 2 |
| $T2C_5=$ | 10 | 1.0% | 11 | 12 | 9 |
| $C_2C_5=$ | 5 | <1.0% | 5 | 5 | 7 |
| $2M2C_4=$ | 65 | 88.0% | 62 | 61 | 60 |

(a) Contains also 1% butenes and 2% i-$C_5$.

Although the $C_4$ olefin distribution of Table 2 was not determined, it is reasonable to expect isomer formation and the isomers to be at equilibrium, which is 6% 1-butene, 37% 2-butenes, and 57% isobutene. This assumption is predictable in view of the work of F. D. Rossini, "The Science of Petroleum", Vol. V, Part I, pp. 153—181, Oxford University Press, 1950.

Under the conditions of operation herein defined, substantially any material inert to the olefination reactions desired to be promoted may be employed as the diluent material mixed with the olefin charge. Some diluents suitable for the process include nitrogen, carbon monoxide, carbon dioxide, helium, paraffins (component of the charge) and hydrogen.

In the combination process of this invention, an olefinic charge material available from any source and economically recovered or a light olefinic naphtha product of thermal or catalytic cracking is contacted with the acidic form of the crystalline zeolite catalyst herein identified at a temperature in the range of from about 400°F (204°C) to about 600°F (316°C), preferably from about 450°F (232°C) to about 550°F (288°C), at a pressure less than atmospheric pressure, olefin pressure preferably 27.6 kPa (4 psia) or less, and an olefin feed at a weight hourly space velocity in the range of 0.2 to 5 for a fixed catalyst bed or the equivalent contact time if a fluidized catalyst system is used. The catalyst may be used as a fixed bed of catalyst, a moving catalyst or a fluidized catalyst system may be used. The olefination product stream comprising a mixture of olefinic hydrocarbons predominantly $C_4$ to $C_7$ olefins

7

and primarily $C_4$ to $C_5$ olefins comprising olefin isomers with minor amounts of lower and higher boiling components, and very little, if any, aromatics are recovered as a product of the acidic zeolite catalyst olefination step.

All or only a portion of the zeolite catalyst conversion product such as the $C_4$ and $C_5$ olefin product above identified is then converted in the presence of methanol and a solid acidic catalyst to form high octane ether products as herein discussed.

Some examples pertinent to the olefin producing step of the invention are provided below.

Example 4—Hydrogen/propylene mixtures

The results obtained by passing propylene alone and mixed with 1, 5 and 10 volumes of hydrogen over acid H-ZSM-5 extrudate (35% alumina binder) are provided in Table 4. (Propylene partial pressures of 101.3, 51, 17.2 and 9 kPa [psia's 14.7, 7.4, 2.5, 1.3, respectively] were obtained with this hydrogen dilution).

Example 5—Nitrogen/propylene mixtures

Table 5 presents the results obtained by mixing 5 and 10 volumes of nitrogen with propylene (propylene partial pressures 17.2 and 9 kPa [psia's 2.5 and 1.3]).

Example 6—Propane/propylene mixtures

Table 6 presents the results obtained by mixing 0.7, 5 and 10 volumes of propane with propylene (propylene partial pressures 59.3, 17.2 and 9 kPa [psia's 8.6, 2.5 and 1.3, respectively]).

Example 7—Nitrogen/propane/propylene/butylene mixtures

Table 7 presents the results obtained with the above mixtures providing olefin partial pressures of 20, 10.3 and 6.9 kPa (2.9, 1.5 and 1.0 psia, respectively).

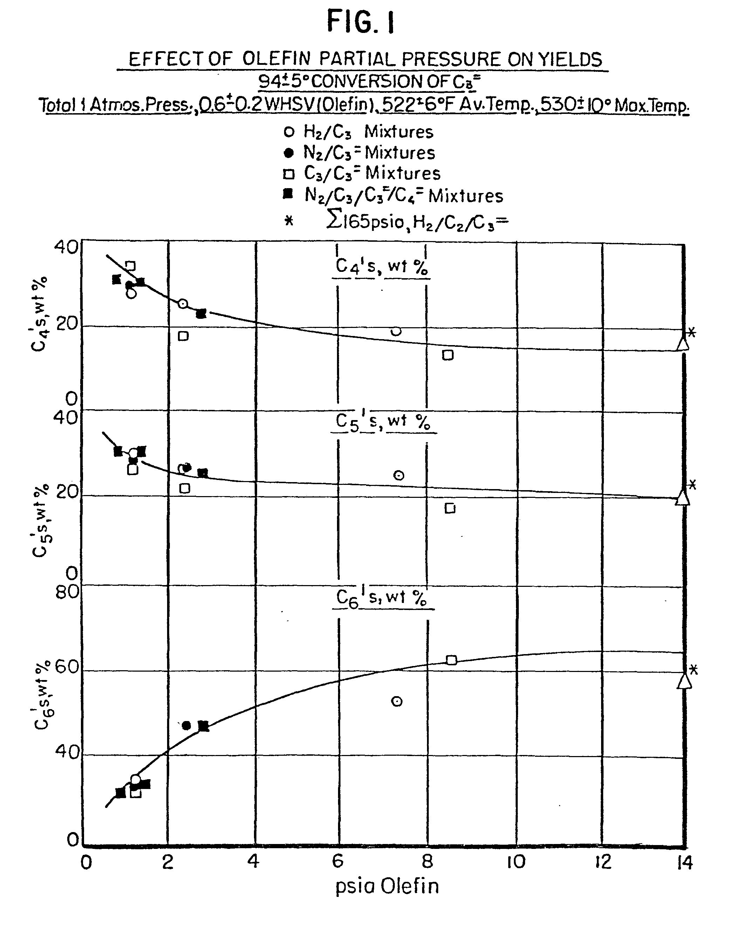

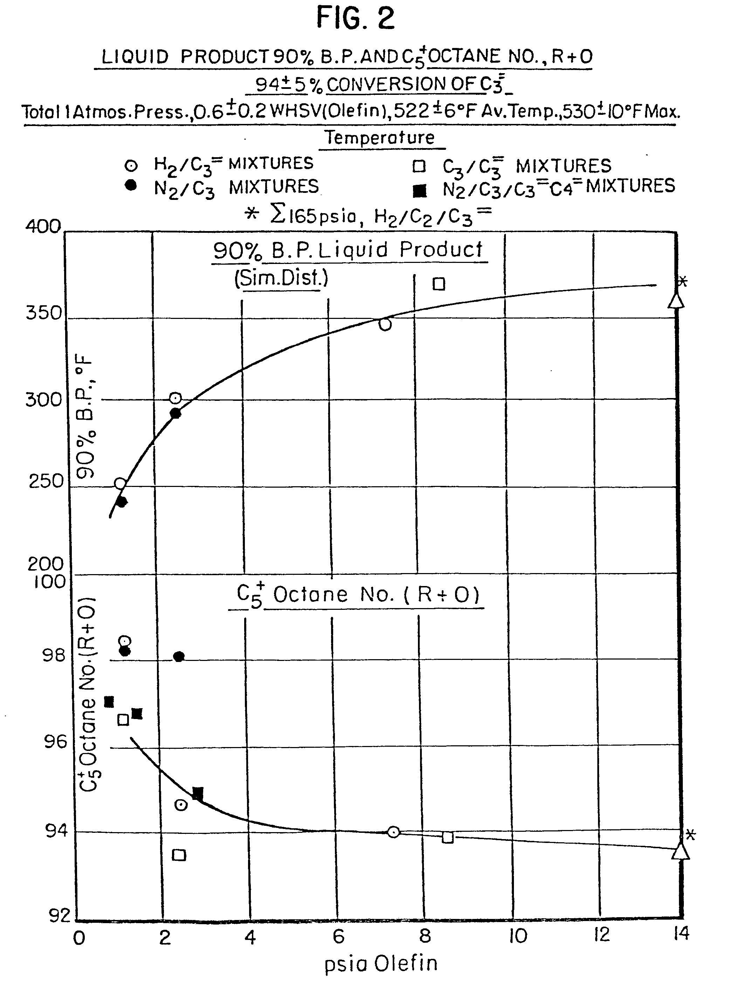

The effect of olefin partial pressure on yields using data from the above four examples is shown in Figure 1. A marked shift to lighter carbon number product occurs at olefin pressures of below about 20.7 kPa (3 psia). The result is a lower boiling range and a higher octane number product (Figure 2).

TABLE 4

Hydrogen/propylene mixtures

| Mole ratio, $H_2/C_3=$ | 0/1 | 1/1 | 5/1 | 10/1 | 10/1/0.7 $(H_2/C_3=/C_3)$ |
|---|---|---|---|---|---|
| Total pressure, kPa | 101.3 | 101.3 | 101.3 | 101.3 | 1138 |
| (psia) | (14.7) | (14.7) | (14.7) | (14.7) | (165) |
| Olefin partial pressure, | | | | | |
| psia | 14.7 | 7.4 | 2.5 | 1.3 | 14.1 |
| kPa | 101.3 | 51 | 17.2 | 9 | 97.2 |
| Temperature, °F, | | | | | |
| Average | 524 (273°C) | 529 (276°C) | 521 (272°C) | 529 (276°C) | 499 (259°C) |
| Maximum | 529 (276°C) | 540 (282°C) | 526 (274°C) | 533 (278°C) | 502 (261°C) |
| WHSV (olefin) | 0.56 | 0.59 | 0.48 | 0.62 | 0.34 |
| Olefin conversion wt.% | 99 | 98 | 95 | 93 | 97 |
| Catalyst age, days | 9 | 18 | 4 | 17 | 3 (propane free) |
| Yields, wt.% | | | | | |
| $C_1+C_2$ | — | 0.1 | 0.2 | — | 0.1 |
| $C_3$'s, total | 2.0 | 3.1 | 7.8 | 8.1 | 2.6 |
| $C_3=$ | 1.0 | 2.3 | 5.4 | 7.1 | 2.6 |
| $C_3$ | 1.0 | 0.8 | 2.4 | 1.0 | — |
| $C_4$'s total | 8.6 | 18.2 | 24.4 | 27.9 | 16.2 |
| i-$C_4$ | 1.0 | 1.2 | 3.5 | 1.1 | 2.0 |
| $C_4=$ | 7.3 | 16.7 | 20.1 | 26.7 | 12.0 |
| n-$C_4$ | 0.3 | 0.3 | 0.8 | 0.1 | 1.6 |
| $C_5$'s total | 19.0 | 25.8 | 26.7 | 29.7 | 21.7 |
| i-$C_5$ | 1.5 | 1.5 | 2.8 | 0.8 | 1.3 |
| $C_5=$ | 17.1 | 24.0 | 23.0 | 28.6 | 19.0 |
| n-$C_5$ | 0.4 | 0.3 | 0.9 | 0.3 | 1.4 |
| $C_6$ | 70.4 | 50.8 | 40.9 | 34.3 | 59.4 |
| | 100.0 | 100.0 | 100.0 | 100.0 | 100.0 |
| $C_6+$ composition, wt.% | | | | | |
| P | 13 | 11 | 17 | 5 | 10 |
| O | 87 | 89 | 81 | 95 | 89 |
| N | <1 | <1 | 1 | <1 | <1 |
| A | <1 | <1 | 1 | <1 | 1 |
| Liquid product 90% B.P., °F, (Sim. Dist.) | 373 (189°C) | 343 (173°C) | 301 (149°C) | 250 (121°C) | 363 (184°C) |
| Liquid product O.N., R+0 | 93.4 | 94.3 | 95.0 | 98.4 | 93.8 |
| $C_5+$ O.N., R+0 | 93.0 | 94.1 | 94.6 | 98.4 | 93.7 |
| Specific gravity | 0.7255 | 0.7142 | 0.7051 | 0.7030 | 0.7210 |

TABLE 5

Nitrogen/propylene mixtures

| Mole ratio, $N_2/C_3$= | 0/1 | | 5/1 | | 10/1 | |
|---|---|---|---|---|---|---|
| Total pressure, kPa | 101.3 | | 101.3 | | 101.3 | |
| (psia) | 14.7 | | 14.7 | | 14.7 | |
| Olefin partial pressure, psia | 14.7 | | 2.5 | | 1.3 | |
| kPa | 101.3 | | 17.2 | | 9 | |
| Temperature, °F, | | | | | | |
| Average | 524 (273°C) | 522 (272°C) | 523 (273°C) | 526 (274°C) | 452 (233°C) | 410 (210°C) |
| Maximum | 528 (276°C) | 527 (275°C) | 527 (275°C) | 528 (276°C) | 477 (247°C) | 412 (211°C) |
| WHSV (Olefin) | 0.56 | 0.56 | 0.65 | 0.88 | 0.64 | 0.63 |
| Olefin conversion, wt.% | 99 | 95 | 91 | 89 | 82 | 14 |
| Catalyst age, days | 8 | 7 | 15 | 9 | 5 | 6 |
| Yield, wt.% | | | | | | |
| $C_1+C_2$ | — | — | — | — | — | — |
| $C_3$'s total | 2.0 | 6.2 | 9.3 | 12.2 | 18.8 | 87.1 |
| =$C_3$= | 1.0 | 5.2 | 8.7 | 11.5 | 18.2 | 86.4 |
| $C_3$ | 1.0 | 1.0 | 0.6 | 0.7 | 0.6 | 0.7 |
| $C_4$'s total | 8.6 | 19.8 | 29.2 | 27.2 | 20.7 | 1.3 |
| i-$C_4$ | 1.0 | 2.2 | 1.0 | 0.9 | 0.8 | — |
| $C_4$= | 7.3 | 17.2 | 28.1 | 26.2 | 19.9 | 1.3 |
| n-$C_4$ | 0.3 | 0.4 | 0.1 | 0.1 | — | — |
| $C_5$'s, total | 19.0 | 27.1 | 29.1 | 33.0 | 21.2 | 1.8 |
| i-$C_5$ | 1.5 | 2.0 | 0.5 | 0.5 | 0.2 | 0.1 |
| $C_5$= | 17.1 | 24.5 | 27.5 | 32.4 | 21.0 | 1.7 |
| n-$C_5$ | 0.4 | 0.6 | 0.1 | 0.1 | — | — |
| $C_6$+ | 70.4 | 46.9 | 33.4 | 27.6 | 39.3 | 10.0 |
| | 100.0 | 100.0 | 100.0 | 100.0 | 100.0 | 100.0 |
| $C_6$+ composition, wt.% | | | | | | |
| P | 13 | 15 | 4 | 5 | 7 | — |
| O | 87 | 85 | 96 | 95 | 93 | — |
| N | <1 | <1 | <1 | <1 | <1 | — |
| A | <1 | <1 | <1 | <1 | <1 | — |
| Liquid product, 90% B.P., °F. (Sim. Dist.) | 373 (189°C) | 292 (144°C) | 239 (115°C) | — | 291 (144°C) | 340 (171°C) |
| Liquid product O.N., R+0 | 93.4 | 98.2 | 98.5 | 99.9 | 93.8 | — |
| $C_5$+O.N., R+0 | 93.0 | 98.0 | 98.3 | 99.6 | 93.6 | — |
| Specific gravity | 0.7255 | 0.7080 | 0.7042 | 0.6958 | 0.7145 | 0.7332 |

TABLE 6

| Mole ratio, $C_3/C_3=$ | Propane/propylene mixtures | | | |
|---|---|---|---|---|
| | 0.1 | 0.71/1 | 5/1 | 10/1 |
| Total pressure, | | | | |
| (psia) | (14.7) | (14.7) | (14.7) | (14.7) |
| kPa | 101.3 | 101.3 | 101.3 | 101.3 |
| Olefin partial pressure, | | | | |
| psia | 14.7 | 8.6 | 2.5 | 1.3 |
| kPa | 101.3 | 59.3 | 17.2 | 9 |
| Temperature, °F, | | | | |
| Average | 524 (273°C) | 516 (269°C) | 520 (271°C) | 520 (271°C) |
| Maximum | 528 (276°C) | 519 (271°C) | 526 (274°C) | 523 (273°C) |
| WHVS (Olefin) | 0.56 | 0.80 | 0.44 | 0.50 |
| Olefin conversion, wt% | 99 | 97 | 94 | 94 |
| Catalyst age, days | 8 | 14 | 10 | 9 |
| Yields, wt.% | | | (Propane free) | (Propane free) |
| $C_1+C_2$ | — | 0.1 | 0.6 | — |
| $C_3$'s, total | 2.0 | 6.6 | 5.6 | 6.1 |
| $C_3=$ | 1.0 | 2.9 | 5.6 | 6.1 |
| $C_3$ | 1.0 | 3.7 | — | — |
| $C_4$'s total | 8.6 | 13.7 | 17.1 | 34.0 |
| $i-C_4$ | 1.0 | 1.0 | 3.0 | 8.2 |
| $C_4=$ | 7.3 | 12.7 | 14.0 | 25.7 |
| $n-C_4$ | 0.3 | — | 0.1 | 0.1 |
| $C_5$'s, total | 19.0 | 16.9 | 21.3 | 27.0 |
| $i-C_5$ | 1.5 | 0.7 | 2.3 | 1.4 |
| $C_5=$ | 17.1 | 16.2 | 18.7 | 25.4 |
| $n-C_5$ | 0.4 | — | 0.3 | 0.2 |
| $C_6+$ | 70.4 | 62.7 | 55.4 | 32.9 |
| | 100.0 | 100.0 | 100.0 | 100.0 |
| $C_6+$ composition, wt.% | | | | |
| P | 13 | 9 | 16 | 7 |
| O | 87 | 90 | 83 | 93 |
| N | <1 | <1 | <1 | <1 |
| A | <1 | 1 | 1 | <1 |
| Liquid product 90% B.P., °F | | | | |
| (Sim. Dist.) | 373 (189°C) | 368 (187°C) | 369 (187°C) | — |
| Liquid product O.N., R+O | 93.4 | 93.5 | 93.6 | 96.7 |
| $C_5+$O.N., R+O | 93.0 | 93.9 | 93.6 | 96.6 |
| Specific gravity | 0.7255 | 0.7287 | 0.7176 | 0.7035 |

## TABLE 7

### Nitrogen/propane/propylene/butylene mixtures

| Ratio, $N_2/C_3/C_3=/C_4=$ | 0/6.6/1/0.7 | 8.5/6.6/1/0.7 | 17/6.6/1/0.7 |
|---|---|---|---|
| Total pressure | | | |
| kPa | 101.3 | 101.3 | 101.3 |
| (psia) | (14.7) | (14.7) | (14.7) |
| Olefin partial pressure, | | | |
| psia | 2.9 | 1.5 | 1.0 |
| kPa | 20 | 10.3 | 6.9 |
| Temperature, °F, | | | |
| Average | 521 (272°C) | 522 (272°C) | 521 (272°C) |
| Maximum | 525 (274°C) | 530 (277°C) | 529 (276°C) |
| WHSV (olefin) | 0.40 | 0.46 | 0.44 |
| Olefin conversion, wt%, | | | |
| $C_3=$ | 93 | 90 | 88 |
| $C_4=$ | 50 | 31 | 29 |
| Total | 76 | 67 | 65 |
| Catalyst, age, days | 11 | 12 | 12.5 |
| Yields, wt.% (Propane free) | | | |
| $C_1+C_2$ | — | — | — |
| $C_3$'s total | 4.4 | 6.2 | 7.5 |
| $C_3=$ | 4.4 | 6.2 | 7.5 |
| $C_3$ | — | — | — |
| $C_4$'s Total | 23.1 | 30.0 | 30.5 |
| i-$C_4$ | 3.5 | 2.9 | 2.7 |
| $C_4=$ | 19.5 | 26.6 | 27.7 |
| n-$C_4$ | 0.1 | 0.5 | 0.1 |
| $C_5$'s total | 24.9 | 30.3 | 30.2 |
| i-$C_5$ | 2.2 | 2.8 | 2.0 |
| $C_5=$ | 22.4 | 27.3 | 28.1 |
| n-$C_5$ | 0.3 | 0.2 | 0.1 |
| $C_6=$ | 47.6 | 33.5 | 31.8 |
| | 100.0 | 100.0 | 100.0 |
| $C_6+$ composition, wt.% | | | |
| P | 14 | 9 | 9 |
| O | 86 | 90 | 91 |
| N | <1 | <1 | <1 |
| A | <1 | 1 | <1 |
| Liquid product 90% B.P., °F | | | |
| (Sim. Dist.) | 333 (167°C) | — | — |
| Liquid product O.N., R+O | 94.9 | 96.8 | 97.1 |
| $C_5$+O.N., R+O | 94.9 | 96.7 | 97.0 |
| Specific gravity | 0.7119 | 0.7005 | 0.7000 |

The effect of controlling olefin feed partial pressure on product yields is provided by the data of the above tables and descriptively shown by Figure 1. A marked shift to lighter carbon number product is observed to occur at olefin pressures below about 27.6 kPa (4 psia). The result is a low boiling range product of relatively high octane number, as shown by the plot of data forming Figure 2. It is observed that butenes increase with propylene as the feed, but with a mixed $C_3$—$C_4$ olefin feed, some butylene also olefinates without loss of product quality. Yields of $C_4$'s, $C_5$'s and $C_6$ plus product change only slightly as the olefin partial pressure is decreased from 96.5 kPa (14 psia) to about 27.6 kPa (4 psia). However, between 20.7 kPa (3 psia) and 6.9 kPa (1 psia), $C_4$'s and $C_5$'s rise relatively sharply at the expense of $C_6$ plus material. Whether hydrogen, nitrogen or propane is used as a diluent does not appear to be critical. A loss in $C_5$ plus yield due to an increase of $C_4$'s with propylene as the charge can be offset by including butylenes in the olefin charge.

In Figure 2 are plotted liquid product 90% boiling points and $C_5$ plus R+O octane numbers v. olefin partial pressure. It will be observed that beginning about 27.6 kPa (4 psia) and more particularly about 20.7 kPa (3 psia) olefin partial pressure, the curve representing the 90% boiling point drops more rapidly and the $C_5$ plus octane number product curve increases rather sharply beginning at 27.6 kPa (4 psia) from about 94 to 97, with and without butylene in the charge.

# 0 026 041

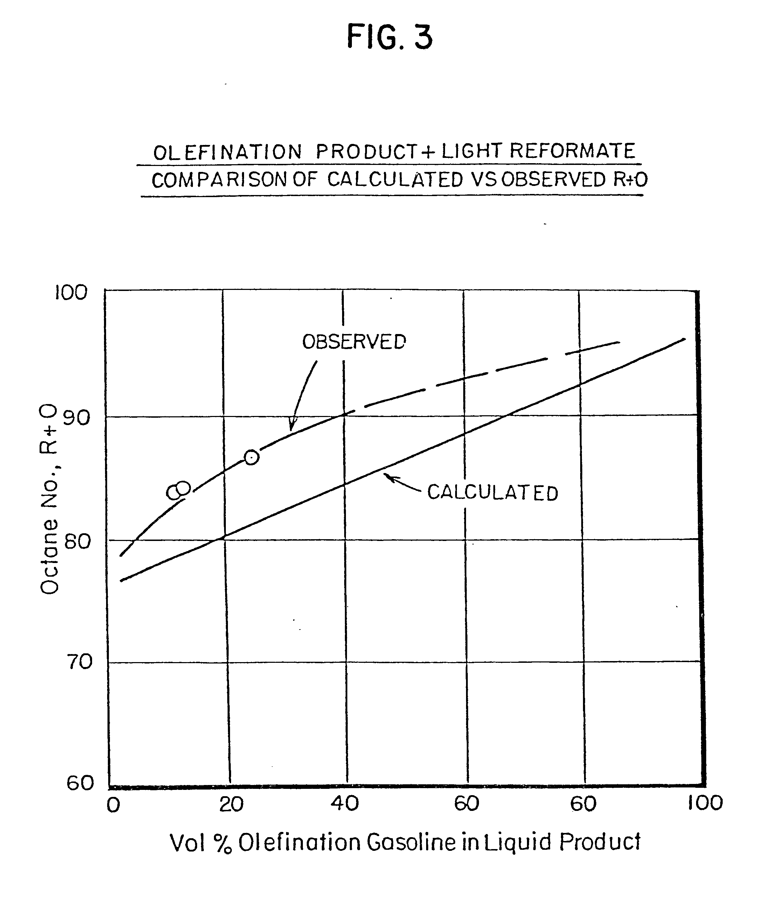

In Table 8 and Figure 3 are presented the results of data obtained using a stable liquid (stable at operating conditions) as a diluent for the olefination conversion operation.

In this example, the stable liquid is a $C_6$ to 200°F (93°C) reformate product which contains 60—80% paraffins and the remaining material comprises benzene and toluene. A part of these aromatics may be alkylated by the olefin, but that doesn't affect their properties as a diluent. Therefore, paraffins, aromatics, and naphthenes can be considered stable liquid diluents under the particular olefination conditions. Note that the carbon number distribution of olefinic product is similar for the nitrogen and reformate diluents. Figure 3 illustrates the remarkably high octane values obtained by the combination of the light olefination component with light reformate. For example, a 50/50 blend of light reformate octane value of about 76 R+0 and olefination product provides a product mixture of 92 (R+0) octane number.

TABLE 8

| Diluent | None | $N_2$ | Reformate | $C_6$-200°F |
|---|---|---|---|---|
| Diluent/$C_3$=, mole | — | 10/1 | 3/1 | 1.5/1 |
| Temp. max., °F | 530 (277°C) | 530 (277°C) | 540 (282°C) | 540 (282°C) |
| Olefin pressure, Atm. (kPa) | 1 (101) | 0.1 (10) | 0.25 (25) | 0.4 (40.5) |
| Olefin conversion, wt.% | 99 | 91 | 96 | 93 |
| Yields, wt.% total charge | | | | |
| $C_1$+$C_2$+Propane | <1 | <1 | <1 | <1 |
| $C_4$ | 9 | 29 | 3 | 5 |
| $C_5$+ | 90 | 63 | 96 | 93 |
| R+0, $C_5$+ | 93 | 98 | 84 (see Fig. 3) | 87 |
| Sim. Dist., 90% OH at °F | 375 (191°C) | 240 (116°C) | 230 (110°C) | 245 (118°C) |
| Olefin distribution in $C_5$+ | | | | |
| $C_5$= | 17 | 37 | 19 | 22 |
| $C_6$= | 15 | 23 | 24 | 22 |
| $C_7$= | 17 | 20 | 25 | 20 |
| $C_8$= | 15 | 10 | 10 | 9 |
| $C_9$+ | 21 | 7 | 4 | 3 |

The olefination product of the crystalline zeolite restructuring operation above identified and specifically discussed is upgraded to an even higher octane product by reacting olefin isomers therein particularly comprising isobutylene and isoamylene with methanol to form ether products thereof and particularly methyl tertiary butyl ether (MTBE) and tertiary amyl methyl ether (TAME).

The reaction of methanol with isobutylene and isoamylenes at moderate conditions with a resin catalyst is known technology as provided by R. W. Reynolds et al. The Oil and Gas Journal, June 16, 1975, and S. Pecci and T. Floris, Hydrocarbon Processing, Dec. 1977. An article entitled "MTBE and TAME—a good octane boosting combo" by J. D. Chase et al, The Oil and Gas Journal, April 9, 1979, pages 149—152, discusses the technology.

The processing combination of the present invention embodies the known technology of forming high octane ethers by reacting methanol with either isobutylene or isoamylene in the presence of solid acidic catalysts in combination with the olefination technology and processing combination of the present invention.

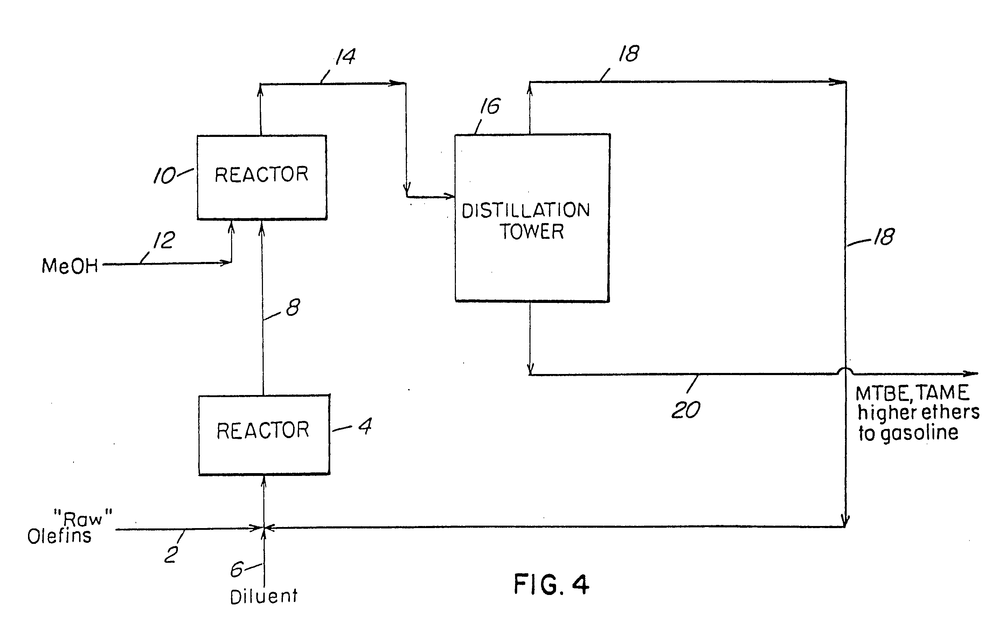

The drawing, Figure 4, is a diagrammatic sketch in elevation of the processing combination of this invention comprising the formation of an olefinic product such as $C_4$ and $C_5$ tertiary isoolefins and the conversion thereof to higher octane ethers.

Referring now to the drawing, Figure 4, by way of example, an olefin feed comprising $C_{10}$ and lower boiling olefins such as provided by thermal and catalytic naphthas and/or other combination olefin refining product streams are charged to the process by conduit 2 and thence to reactor 4. A diluent material considered generally inert to the process is charged by conduit 6. In reactor 4, the olefins are rearranged as by olefination reactions as herein described at a temperature below 600°F (316°C) and more usually the temperature is below about 550°F (288°C). The reaction is effected at olefin subatmospheric pressure by contact with an acid ZSM-5 crystalline zeolite to provide a product rich in $C_4$ and $C_5$ olefins and comprising isobutylene and isoamylene.

The product of the zeolite catalyst operation in reactor 4 is withdrawn for further treatment as herein discussed. In one embodiment, it is contemplated, charging the total effluent of olefination reactor 4 in contact with a solid acidic catalyst for reaction with an alcohol to form high octane ethers. In another embodiment, the zeolite catalyst olefination effluent is separated in a zone not shown to recover primarily $C_4$ and $C_5$ boiling components and particularly olefin isomers thereof from other olefins and particularly from $C_6$ and higher boiling components. The separated $C_6$ and higher boiling components are recycled to the zeolite catalyst olefination conversion reactor stage 4 by means not shown for further conversion therein to desired isolefins. Whichever embodiment is relied upon, the

13

product of olefination reactor 4, comprising $C_4$ and $C_5$ olefins, is passed by conduit 8 to ether forming reactor 10. A lower alcohol, preferably methanol, is also charged to ether forming reactor 16 by conduit 12. In the ether forming reactor, the charged methanol and olefins comprising isobutylene and tertiary isoamylenes are reacted in the presence of a solid acidic catalyst to form TAME and MTBE. In a particular operation, a commercially available sulfonic acid resin catalyst may be employed.

The product of reactor 10 and comprising high octane ethers, unreacted olefins and methanol are passed by conduit 14 to a separation zone 16. Separation zone 16 is maintained at a desired pressure, normally atmospheric pressure, and a temperature within the range of 80 (27°C) to 125°F (52°C), depending on the separation desired. In one arrangement, $C_5$ and lower boiling unreacted olefins along with unreacted methanol are separated and withdrawn by conduit 18 for recycle to the olefination reactor 4. In reactor 4, the unconverted methanol and olefins recycled are converted and/or restructured to form desired olefin product.

On the other hand, it may be desirable to retain in the separation effected in separator 16 the $C_5+$ unreacted olefins with the formed high octane ether product. In this separation and recovery arrangement, the unreacted $C_5+$ olefins may also be separated from the high octane ethers and thereafter blended in pool gasoline. Thus, depending on the separating temperature and pressure conditions relied upon, the high octane ether product (TAME and MTBE) separated in zone 16 either with or without $C_5$ plus olefins is withdrawn by conduit 20 as a primary product of the combination process. On the other hand, the separation may be made to recover $C_6$ plus unreacted olefins with the ether product rather than the $C_5$ plus unreacted olefins. Therefore either a $C_4$ or a $C_5$ minus product of ether formation may be separated and recycled to olefination zone 4.

MTBE and TAME are known to be high octane ethers. The above referred to article by J. D. Chase et al, Oil and Gas Journal, April 9, 1979, clearly identifies the advantages one can achieve by using these materials to alter gasoline octane. Thus, it is clear that, where a shortage of isobutylene and isoamylene exists and low octane olefins abound, the processing combination of the present invention contributes measurably to reducing the problems associated with low octane gasoline product. The octane blending number of MTBE when 10% is added to a base fuel R+0=91 is about 120. For a base fuel with a motor rating (M+0) of 83 octane the blending value of MTBE at the 10% level is about 103. On the other hand, for an (R+0) of 95 octane fuel the blending value of 10% MTBE is about 114, and for an (M+0) of 84 octane the 10% blending value is about 100.

Having thus generally described the method and concepts of the present invention and presented specific examples in support thereof, it is to be understood that no undue restrictions are to be imposed by reason thereof except as defined by the following claims.

## Claims

1. A method for producing gasoline boiling range high octane products from a mixture of olefins comprising up to $C_{10}$ olefins which comprises, contacting a mixed olefin feed with an acid crystalline zeolite catalyst characterized by a pore opening of at least $5 \times 10^{-10}$m, a silica to alumina ratio of at least 12 and a constraint index within the range of 1 to 12 at an olefin pressure below $5.1 \times 10^4$ Pa (0.5 atmosphere) and a temperature selected from within the range of 400°F (204°C) to 600°F (316°C), and recovering an olefinic product enriched with $C_4$ and $C_5$ olefins comprising olefin isomers.

2. The method of claim 1 wherein the olefin feed is contacted with said crystalline zeolite at a temperature within the range of 500°F (260°C) to 550°F (288°C) at an olefin pressure not above $3 \times 10^4$ Pa (0.3 atmosphere) and sufficient to obtain 95% conversion to a higher octane product comprising less than 2% aromatics.

3. The method of claim 1 wherein a diluent material substantially inert to the restructuring of the olefins charged is employed with said olefin feed to maintain the partial pressure of the olefin feed below $5.1 \times 10^4$ Pa (0.5 atmosphere).

4. The method of claim 1 wherein the olefin feed comprises a mixture of $C_2$ to $C_{10}$ carbon compounds.

5. The method of claim 1 wherein the olefin feed is mixed with one or more of hydrogen, propane and nitrogen.

6. The method of claim 1 wherein the olefinic product comprises isobutylene and isopentenes which materials are reacted with an alcohol by contact with an acidic catalyst to form high octane ethers.

7. A method for producing high octane ether products comprising tertiary amyl methyl ether and methyl tertiary butyl ether from a $C_2$ to $C_{10}$ olefin feed mixture which comprises, reacting the $C_2$ to $C_{10}$ olefin feed under temperature and pressure conditions in the presence of an acid zeolite catalyst providing a pore opening of at least $5 \times 10^{-10}$ m, a silica-alumina ratio of at least 12 and a constraint index within the range of 1 to 12 to form an olefin product richer in $C_4$ and $C_5$ olefins and comprising olefin isomers of isobytylene and isopentenes, reacting at least the olefin isomers with a lower alcohol in the presence of an acid catalyst under temperature and pressure conditions promoting the formation of the high octane ethers above identified, separating the formed ethers from unreacted $C_4$ minus

14

## 0 026 041

olefins and alcohol and recovering the separated ethers, and recycling the separated $C_4$ minus olefins and alcohol for contact with the zeolite catalyst above identified.

8. The method of claim 7 wherein $C_5$ plus olefins are recovered with the ether product.

9. The method of claim 7 wherein olefins boiling below $C_6$ olefins are separated from the ether product and recycled to the zeolite catalyst conversion step.

10. The method of claim 7 wherein the formation of ethers from $C_4$ and $C_5$ olefin isomers is accomplished with methanol and an acidic resin catalyst.

11. The method of claim 7 wherein the olefin product of the zeolite catalyst conversion operation is separated to recover particularly $C_4$ to $C_7$ olefins which are thereafter reacted with methanol in the presence of a solid acidic catalyst to form the ether products desired and unreacted olefins are separated from the ether product to provide separate streams thereof for use in blending to form desired gasoline product.

12. A method for producing high octane ethers and olefins from a mixture of $C_2$ to $C_{10}$ olefins which comprises, converting the $C_2$ to $C_{10}$ olefins in the presence of a special zeolite catalyst by olefination thereof to a product enriched in $C_4$ to $C_7$ olefins, reacting the $C_4$ to $C_7$ olefins thus produced with methanol and in the presence of an acid catalyst to form ethers of isobutylene and isopentenes, separating the ether product and $C_6$ plus olefins from unreacted $C_5$ minus olefins and methanol, and recycling the $C_5$ minus olefins with methanol to the special zeolite catalyst olefination operation, said special zeolite catalyst comprising a crystalline zeolite characterized by a pore opening of at least $5 \times 10^{-10}$ m, a silica to alumina ratio of at least 12 and a constraint index within the range of 1 to 12.

### Patentansprüche

1. Verfahren zur Herstellung von Hochoctanprodukten im Benzinsiedebereich aus einem Gemisch von Olefinen, enthaltend bis zu $C_{10}$-Olefine, dadurch gekennzeichnet, daß ein gemischtes Olefinzufuhrmaterial mit einem sauren kristallinen Zeolithkatalysator mit einer Porenöffnung von wenigstens $5 \times 10^{-10}$m, einem Siliciumdioxid/Aluminiumoxid-Verhältnis von wenigstens 12 und einem Zwangsindex innerhalb des Bereiches von 1 bis 12 bei einem Olefindruck unterhalb $5,1 \times 10^4$ Pa (0,5 at) und einer Temperatur aus dem Bereich von 400°F (204°C) bis 600°F (316°C) in Berührung gebracht und ein olefinisches Produkt gewonnen wird, welches mit $C_4$- und $C_5$-Olefinen angereichert ist, enthaltend Olefinisomere.

2. Verfahren nach Anspruch 1, dadurch gekennzeichnet, daß das Olefinzufuhrmaterial mit dem kristallinen Zeolithen bei einer Temperatur innerhalb des Bereiches von 500°F (260°C) bis 550°F (288°C) bei einem Olefindruck nicht oberhalb von $3 \times 10^4$ Pa (0,3 at) und ausreichend zur Erzielung von 95 %iger Umwandlung in ein höheres Octanprodukt, enthaltend weniger als 2% Aromaten, in Berührung gebracht wird.

3. Verfahren nach Anspruch 1, dadurch gekennzeichnet, daß ein Verdünnungsmaterial, welches im wesentlichen inert gegenüber der Restrukturierung der eingebrachten Olefine ist, mit dem Olefinzufuhrmaterial zur Aufrechterhaltung des Partialdruckes des Olefinzufuhrmaterials unterhalb $5,1 \times 10^4$ Pa (0,5 at) verwendet wird.

4. Verfahren nach Anspruch 1, dadurch gekennzeichnet, daß das Olefinzufuhrmaterial ein Gemisch von $C_2$- bis $C_{10}$-Kohlenstoffverbindungen umfaßt.

5. Verfahren nach Anspruch 1, dadurch gekennzeichnet, daß das Olefinzufuhrmaterial mit einer oder mehreren Komponenten aus der Reihe Wasserstoff, Propan und Stickstoff gemischt ist.

6. Verfahren nach Anspruch 1, dadurch gekennzeichnet, daß das olefinische Produkt Isobutylen und Isopentene enthält, welche Materialien mit einem Alkohol durch Berührung mit einem sauren Katalysator zur Bildung von Hochoctanäthern umgesetzt werden.

7. Verfahren zur Herstellung von Hochoctanätherprodukten, enthaltend tert. Amylmethyläther und Methyl-tert. Butyläther, aus einem $C_2$- bis $C_{10}$-Olefinzufuhrmaterialgemisch, dadurch gekennzeichnet, daß das $C_2$- bis $C_{10}$-Olefinzufuhrmaterial unter Temperatur- und Druckbedingungen in Gegenwart eines sauren Zeolithkatalysators, der eine Porenöffnung von wenigstens $5 \times 10^{-10}$ m, ein Siliciumdioxid/Aluminiumoxid-Verhältnis von wenigstens 12 und einen Zwangsindex innerhalb des Bereiches von 1 bis 12 gewährleistet, unter Bildung eines Olefinproduktes, welches reicher an $C_4$- und $C_5$-Olefinen ist und Olefinisomere von Isobutylen und Isopentenen enthält, umgesetzt wird, daß wenigstens die Olefinisomeren mit einem niedrigeren Alkohol in Gegenwart eines sauren Katalysators unter Temperatur- und Druckbedingungen umgesetzt werden, welche die Bildung der Hochoctanäther, wie oben angegeben, fördern, die gebildeten Äther von nichtumgesetzten $C_4$(minus)-Olefinen und Alkohol abgetrennt und die abgetrennten Äther gewonnen werden und die abgetrennten $C_4$(minus)-Olefine und Alkohol zur Berührung mit dem Zeolithkatalysator, wie oben angegeben, wieder in Umlauf gebracht werden.

8. Verfahren nach Anspruch 7, dadurch gekennzeichnet, daß die $C_5$(plus)-Olefine mit dem Ätherprodukt gewonnen werden.

9. Verfahren nach Anspruch 7, dadurch gekennzeichnet, daß die Olefine, welche unterhalb von $C_6$-Olefinen sieden, von dem Ätherprodukt abgetrennt und zur Zeolithkatalysator-Umwandlungsstufe wieder in Umlauf gebracht werden.

15

10. Verfahren nach Anspruch 7, dadurch gekennzeichnet, daß die Bildung von Äthern aus $C_4$- und $C_5$-Olefinisomeren mit Methanol und einem sauren Harzkatalysator erzielt wird.

11. Verfahren nach Anspruch 7, dadurch gekennzeichnet, daß das Olefinprodukt des Zeolithkatalysator-Umwandlungsvorganges zur Gewinnung insbesondere von $C_4$- bis $C_7$-Olefinen abgetrennt wird, die denach mit Methanol in Gegenwart eines festen sauren Katalysators unter Bildung der gewünschten Ätherprodukte umgesetzt werden, und nichtumgesetzte Olefine aus dem Ätherprodukt unter Bildung von gesonderten Strömen davon zur Anwendung in Vermischung zur Bildung von gewünschtem Benzinprodukt abgetrennt werden.

12. Verfahren zur Herstellung von Hochoctanäthern und Olefinen aus einem Gemisch von $C_2$- bis $C_{10}$-Olefinen, dadurch gekennzeichnet, daß die $C_2$- bis $C_{10}$-Olefine in Gegenwart eines besonderen Zeolithkatalysators durch Olefinisierung in ein Produkt, welches mit $C_4$- bis $C_7$-Olefinen angereichert ist, umgesetzt werden, die so erzeugen $C_4$-bis $C_7$-Olefine mit Methanol und in Gegenwart eines sauren Katalysators unter Bildung von Äthern von Isobutylen und Isopentenen umgesetzt werden, das Ätherprodukt und $C_6$(plus)-Olefine von nichtumgesetzten $C_5$(minus)-Olefinen und Methanol abgetrennt werden und die $C_5$(minus)-Olefine mit Methanol zu dem Olefinisierungsvorgang mit dem besonderen Zeolithkatalysator wieder in Umlauf gebracht werden und der besondere Zeolithkatalysator einen kristallinen Zeolithen enthält, welcher eine Porenöffnung von wenigstens $5\times10^{-10}$ m, ein Siliciumdioxid/Aluminiumoxid-Verhältnis von wenigstens 12 und einen Zwangsindex innerhalb des Bereiches von 1 bis 12 hat.

## Revendications

1. Une méthode pour produire des produits octaniques élevés dans le domaine d'ébullition de l'essence à partir d'un mélange d'oléfines comportant jusqu'à des oléfines en $C_{10}$, qui consiste à mettre en contact une charge oléfinique mixte avec un catalyseur à base de zéolite cristalline acide, caractérisée par une ouverture des pores d'au moins $5\times10^{-10}$m, un rapport de silice à alumine d'au moins 12 un indice de contrainte dans la plage de 1 à 12 pour une pression d'oléfine inférieure à $5,1\times10^4$ Pa (0,5 atmosphère) et une température choisie dans la plage de 204°C à 316°C, et à récupérer un produit oléfinique enrichi en oléfines en $C_4$ et $C_5$ comportant des isomères oléfiniques.

2. La méthode de la revendication 1, dans laquelle la charge d'oléfine est mise en contact avec cette zéolite cristalline à une température dans la plage de 260 à 288°C à une pression d'oléfine pas supérieure à $3\times10^4$ Pa (0,3 atmosphère) et suffisante pour obtenir une conversion à 95% à un produit octanique élevé comportant moins de 2% de produits aromatiques.

3. La méthode de la revendication 1, dans laquelle un matériau diluant pratiquement inerte dans la restructuration des oléfines chargées est utilisé avec cette charge d'oléfine pour maintenir la pression partielle de la charge d'oléfine en dessous de $5,1\times10^4$ Pa (0,5 atmosphère).

4. La méthode de la revendication 1, dans laquelle la charge oléfinique comporte un mélange de composé carboné en $C_2$ à $C_{10}$.

5. La méthode de la revendication 1, dans laquelle la charge oléfinique est mélangée avec de l'hydrogène, du propane et de l'azote, pris seul en en mélange.

6. La méthode de la revendication 1, dans laquelle le produit oléfinique comporte de l'isobutylène et des isopenténes, ces matériaux étant mis à réagir avec un alcool par contact avec un catalyseur acide pour former des éthers octaniques élevées.

7. Une méthode pour produire des produits éthérés octaniques élevés comportant du ter-amylate de méthyle et du ter-butylate de méthyle à partir d'un mélange de charge oléfinique en $C_2$ à $C_{10}$ qui consiste à faire réagir la charge oléfinique en $C_2$ à $C_{10}$ sous des conditions de température et de pression en présence d'un catalyseur à base de zéolite acide fournissant une ouverture de pores d'au moins $5\times10^{-10}$m, un rapport silicealumine d'au moins 12 et un indice de contrainte dans la plage de 1 à 12 pour former un produit oléfinique plus riche en oléfines en $C_4$ et $C_5$ et comportant des isomères oléfiniques d'isobutylène et d'isopentènes, à faire réagir les isomères oléfiniques avec un alcool inférieur en présence d'un catalyseur acide sous des conditions de température et de pression pour favoriser la formation d'éthers octaniques élevés identifiés cidessus, à séparer les éthers formées des oléfines n'ayant pas réagi en $C_4$ et moins et de l'alcool et à récupérer les éthers séparés, et à recycler les oléfines séparées en $C_4$ et moins et l'alcool par mise en contact avec le catalyseur à base de zéolite identifié ci-dessus.

8. La méthode de la revendication 7, dans laquelle les oléfines en $C_5$ et plus sont récupérées avec le produit éthéré.

9. La méthode de la revendication 7, dans laquelle les oléfines en $C_6$ de point d'ébullition inférieur sont séparées du produit éthéré et recyclées au stade de conversion du catalyseur à base de zéolite.

10. La méthode de la revendication 7, dans laquelle la formation des éthers d'isomères d'oléfines en $C_4$ et $C_5$ est réalisée avec du méthanol et un catalyseur à base de résine acide.

11. La méthode de la revendication 7, dans laquelle le produit oléfinique du mode opératoire de conversion à catalyseur à base de zéolite est séparé pour récuper en particulier les oléfines en $C_4$ à $C_7$ qui sont par la suite mises à réagir avec du méthanol en présence d'un catalyseur acide solide pour former les produits éthérés désirés et les oléfines n'ayant pas réagi sont séparées du produit éthéré pour fournir des courants séparés afin d'être utilisées en mélange pour former le produit à base d'essence désiré.

12. Une méthode pour produire des éthers et des oléfines octaniques élevés à partir d'un mélange d'oléfine octanique élevé à partir d'un mélange d'oléfines en $C_2$ à $C_{10}$ qui consiste à convertir les oléfines en $C_2$ à $C_{10}$ en présence d'un catalyseur à base de zéolite spécial par oléfination de ces dernières en un produit enrichi en oléfines en $C_4$ à $C_7$, à faire réagir les oléfines en $C_4$ à $C_7$ ainsi produites avec du méthanol et en présence d'un catalyseur acide pour former des éthers d'isobutylène et d'isopentènes, à séparer le produit éthéré et les oléfines en $C_6$ et plus des oléfines n'ayant pas réagi en $C_5$ et moins et du méthanol, et à recycler les oléfines en $C_5$ et moins avec le méthanol à un mode opératoire d'oléfination à catalyseur à base de zéolite spéciale, ce catalyseur à zéolite spéciale comportant une zéolite cristalline caractérisée par une ouverture des pores d'au moins $5 \times 10^{-10}$m, un rapport de silice à alumine d'au moins 12 et un indice de contrainte dans la plage de 1 à 12.

0 026 041

# FIG. I

## EFFECT OF OLEFIN PARTIAL PRESSURE ON YIELDS
### $94 \pm 5°$ CONVERSION OF $C_3^=$
Total 1 Atmos. Press., $0.6 \pm 0.2$ WHSV (Olefin), $522 \pm 6°F$ Av. Temp., $530 \pm 10°$ Max. Temp.

○ $H_2/C_3$ Mixtures
● $N_2/C_3^=$ Mixtures
□ $C_3/C_3^=$ Mixtures
■ $N_2/C_3/C_3^=/C_4^=$ Mixtures
✳ $\Sigma 165$ psia, $H_2/C_2/C_3^=$

FIG. 2

LIQUID PRODUCT 90% B.P. AND $C_5^+$ OCTANE NO., R + O

$94 \pm 5\%$ CONVERSION OF $C_3^=$

Total 1 Atmos. Press., $0.6 \pm 0.2$ WHSV (Olefin), $522 \pm 6°F$ Av. Temp., $530 \pm 10°F$ Max.

# FIG. 3

OLEFINATION PRODUCT + LIGHT REFORMATE
COMPARISON OF CALCULATED VS OBSERVED R+O

Vol % Olefination Gasoline in Liquid Product

FIG. 4